# FASCICULE DE BREVET EUROPEEN

(11) **EP 1 891 934 B1**
(45) Date de publication et mention de la délivrance du brevet: **19.09.2012**
(21) Numéro de dépôt: 07113833.3
(22) Date de dépôt: 06.08.2007
(51) Int. Cl.: A61K 8/81, A61Q 5/10

(54) **Composition tinctoriale comprenant un colorant d'oxydation et un polymère acrylamide, halogènure de dialkyldiallylammonium et acide carboxylique vinylique à fort taux d'acrylamide.**
Farbzusammensetzung enthaltend einen Oxidationsfarbstoff und ein Polymer aus Acrylamid, Dialkyldiallylammoniumhalogenid und Vinylcarbonsäure mit hohem Acrylamidanteil
Tinctorial composition containing an oxidation dye and a polymer of acrylamide, dialkyldiallylammonium halide and vinyl carboxylic acid with a high acrylamide content.

(30) Priorité: 10.08.2006 FR 0607244
(43) Date de publication de la demande: 27.02.2008
(73) Titulaire: L'Oréal, 75008 Paris (FR)
(72) Inventeur: COTTARD, François, 92400, COURBEVOIE (FR); Deconinck, Gautier, 95210, SAINT GRATIEN (FR)
(74) Mandataire: Casalonga, Axel

(56) Documents cités:
- EP-A- 1 048 290
- EP-A1- 0 522 755
- WO-A-02/45674

## Description

La présente invention a pour objet une composition pour la teinture d'oxydation des fibres kératiniques, et en particulier des fibres kératiniques humaines telles que les cheveux, comprenant, dans un milieu approprié pour la teinture, au moins un colorant d'oxydation et au moins un polymère amphotère particulier.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La demande de brevet EP 1 048 290 décrit une composition pour la teinture d'oxydation des cheveux comprenant un précurseur de colorant d'oxydation, un coupleur et un polymère amphotère comprenant des unités répétitives (a) d'acide acrylique et (b) d'un monomère cationique choisi parmi le chlorure de méthacrylamidopropyl triméthyl ammonium, le chlorure de diméthyl diallyl ammonium et leurs mélanges, le rapport molaire entre les unités (a) et les unités (b) étant supérieur ou égal à 1 / 3. Cette composition permet d'améliorer le dépôt des colorants sur les cheveux et de rendre ainsi la teinture plus efficace.

Le brevet US 2005/0015895 décrit une composition pour la teinture d'oxydation des cheveux comprenant un colorant d'oxydation et un terpolymère amphotère quaternaire comprenant les unités répétitives suivantes : (a) le chlorure de méthacrylamidopropyl triméthyl ammonium ou le chlorure de diméthyl diallyl ammonium, (b) l'acide acrylique ou le méthacrylate de sodium, et (c) l'acrylamide, avec un rapport (a) / (b) supérieur ou égal à 4. Cette composition permet d'obtenir un meilleur effet de conditionnement des fibres capillaire.

La demande de brevet EP 0 522 755 décrit des compositions capillaires essentiellement destinées au shampoing, au conditionnement ou à la fixation des cheveux, et qui comprennent un terpolymère acrylamide / chlorure de diméthyldiallylammonium / acide acrylique dans des proportions pondérales respectives de 1 à 95 % / 5 à 80 % / 1 à 75 %.

Enfin, le brevet FR 2 817 467 décrit une composition pour la teinture d'oxydation des fibres kératiniques comprenant un colorant d'oxydation, un polymère associatif et un polymère à motifs issus d'un monomère de type (i) acrylamide, (ii) halogénure de dialkyldiallylammonium, et (iii) acide carboxylique vinylique. Cette composition permet d'obtenir une application optimale sur les fibres, en particulier elle ne coule pas, sans pour autant altérer les qualités de la coloration.

Toutefois, les compositions de teinture d'oxydation de l'art antérieur présentent certains inconvénients. En particulier, leur utilisation est susceptible de faire apparaître des phénomènes d'électricité statique à la surface des fibres kératiniques, ou d'augmenter de tels phénomènes.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de teinture d'oxydation qui permettent de réduire les phénomènes d'électricité statique après la coloration.

Ce but est atteint avec la présente invention qui a pour objet une composition pour la teinture d'oxydation des fibres kératiniques, et en particulier des fibres kératiniques humaines telles que les cheveux, comprenant, dans un milieu approprié pour la teinture, au moins un colorant d'oxydation et au moins un polymère amphotère comprenant la répétition de :
(i) de 45 à 60 % en moles de un ou plusieurs motifs issus d'un monomère de type acrylamide,
(ii) un ou plusieurs motifs issus d'un monomère de type halogénure de dialkyldiallylammonium, et
(iii) un ou plusieurs motifs issus d'un monomère de type acide carboxylique vinylique,
avec un rapport molaire du taux de motifs issus d'un monomère de type halogénure de dialkyldiallylammonium sur le taux de motifs issus d'un monomère de type acide carboxylique vinylique supérieur ou égal à 1.

La composition conforme à la présente invention permet d'éviter ou de réduire les phénomènes d'électricité statique en surface des cheveux, tout en conservant de bonnes propriétés rhéologiques. En outre, elle permet d'améliorer les propriétés cosmétiques des cheveux, notamment en ce qui concerne le démêlage et le lissage.

Enfin, les propriétés tinctoriales de cette composition sont très satisfaisantes, tant en ce qui concerne la sélectivité que l'intensité de la coloration obtenue.

La présente invention a également pour objet un procédé de teinture des fibres kératiniques, dans lequel une composition conforme à l'invention est appliquée sur les fibres kératiniques en présence d'un agent oxydant pendant un temps suffisant pour développer la coloration désirée.

La présente invention a aussi pour objet un dispositif à plusieurs compartiments pour la mise en oeuvre du procédé conforme à l'invention.

La présente invention a aussi pour objet l'utilisation pour la teinture d'oxydation des fibres kératiniques de la composition conforme à l'invention.

La présente invention a enfin pour objet l'utilisation, dans une composition de teinture d'oxydation, d'un polymère amphotère tel que décrit dans la présente demande, afin d'éviter ou de réduire les phénomènes d'électricité statique en surface des cheveux.

A moins d'une indication différente, les bornes des gammes de valeurs qui sont données dans le cadre de la présente invention sont incluses dans ces gammes.

Selon un mode de réalisation particulier, les motifs issus d'un monomère de type acrylamide du polymère amphotère utile dans le cadre de l'invention sont des motifs de structure (I) suivante : 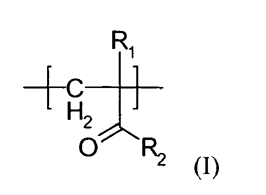 dans laquelle :
- R₁ désigne H ou CH₃,
- R₂ est choisi parmi un radical amino, diméthylamino, tert-butylamino, dodécylamino, ou -NH-CH₂OH.

De préférence, le polymère amphotère de l'invention ne comprend la répétition que d'un seul motif de formule (I).

Le motif issu d'un monomère de type acrylamide de formule (I) dans laquelle R₁ désigne H et R₂ est un radical amino est particulièrement préféré. Il correspond au monomère acrylamide proprement dit.

Selon un autre mode de réalisation particulier de l'invention, les motifs issus d'un monomère de type halogénure de dialkyldiallylammonium du polymère amphotère sont des motifs de structure (II) suivante : 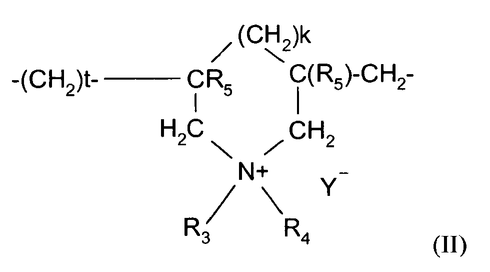 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de préférence 1 à 5 atomes de carbone, un groupement amidoalkyle(C₁-C₄), ou R₃ et R₄ forment conjointement avec l'atome d'azote auquel ils sont rattachés, des groupements hétérocycliques, tels que pipéridinyle ou morpholinyle ; R₃ et R₄ indépendamment l'un de l'autre désignent de préférence un groupement alkyle ayant de 1 à 4 atomes de carbone ;
- Y⁻ est un anion tel que bromure, chlorure, acétate, borate, citrate, tartrate, bisulfate, bisulfite, sulfate, phosphate.

Parmi ces motifs issus d'un monomère de type halogénure de dialkyldiallylammonium, on préfère ceux issus du monomère chlorure de diméthyldiallylammonium pour lesquels R₅ désigne un atome d'hydrogène et R₃ et R₄ désignent un radical méthyle, Y⁻ désignant un anion chlorure.

Selon un mode de réalisation particulier de l'invention, les motifs issus d'un monomère de type acide carboxylique vinylique du polymère amphotère sont choisis parmi les motifs de formule (III) : 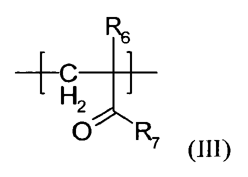 dans laquelle :
- R₆ désigne H ou CH₃,
- R₇ désigne un radical hydroxyle ou un radical -NH-C(CH₃)₂-CH₂-SO₃H.

Les motifs préférés correspondent aux monomères acide acrylique, acide méthacrylique et acide 2-acrylamido 2-méthyl propane sulfonique.

De préférence, le motif issu d'un monomère de type acide carboxylique vinylique est celui issu de l'acide acrylique pour lequel R₆ désigne un atome d'hydrogène et R₇ désigne un radical hydroxyle.

Selon l'invention, le ou les polymères amphotères comprennent de 45 à 60 % en moles de motifs issus d'un monomère de type acrylamide.

De préférence, ils comprennent de 45 à 55 % en moles de motifs issus d'un monomère de type acrylamide.

De préférence, lesdits polymères amphotères comprennent 30 ou moins de 30 % en moles de motifs issus d'un monomère de type acide carboxylique vinylique.

De préférence, ils comprennent de 5 à 30% en moles de motifs issus d'un monomère de type acide carboxylique vinylique, de manière plus préférée de 15 à 25% en moles.

Enfin, le rapport molaire du taux de motifs issus d'un monomère de type halogénure de dialkyldiallylammonium sur le taux de motifs issus d'un monomère de type acide carboxylique vinylique doit être supérieur ou égal à 1. De préférence, ce rapport est compris entre 1 et 2, plus préférentiellement entre 1 et 1,5, encore plus préférentiellement entre 1,1 et 1,3.

Le ou les polymères amphotères peuvent comprendre avantageusement au moins 25 % en moles de motifs issus d'un monomère de type halogénure de dialkyldiallylammonium, préférentiellement de 25 à 30% en moles.

Le ou les polymères amphotères utiles dans le cadre de l'invention peuvent également comprendre des motifs additionnels, différents des motifs issus d'un monomère de type acrylamide, halogénure de dialkyldiallylammonium, et acide carboxylique vinylique, du moment qu'ils comprennent de 45 à 60 % en moles de un ou plusieurs motifs issus d'un monomère de type acrylamide, et présentent un rapport molaire du taux de motifs issus d'un monomère de type halogénure de dialkyldiallylammonium sur le taux de motifs issus d'un monomère de type acide carboxylique vinylique supérieur ou égal à 1.

Comme exemple de polymères à motifs issus d'un monomère de type (i) acrylamide, (ii) halogénure de dialkyldiallylammonium, et (iii) acide carboxylique vinylique, on peut citer notamment les terpolymères acrylamide / chlorure de diméthyldiallylammonium / acide acrylique, répertoriés dans le dictionnaire C.T.F.A. International Cosmetic Ingredient Dictionary, 10ème édition 2004, sous la dénomination "Polyquaternium 39". Les polymères selon l'invention peuvent ainsi être choisis parmi les Polyquaterniums 39 contenant de 45 à 60 % en moles de motifs issus de l'acrylamide, et présentant un rapport molaire du taux de motifs issus du chlorure de diméthyldiallylammonium sur le taux de motifs issus de l'acide acrylique supérieur ou égal à 1, tel que par exemple le produit proposé sous la dénomination MERQUAT 3331 par la société NALCO.

Le polymère amphotère selon l'invention peut être préparé de manière classique, par polymérisation à partir de ses différents monomères, selon des techniques connues de l'homme du métier, et notamment par polymérisation radicalaire.

Le ou les polymères amphotères utiles dans le cadre de l'invention sont généralement présents en quantité comprise entre 0,1 et 10 % en poids, de préférence entre 0,5 et 5 % en poids, et plus particulièrement entre 1 et 4 % en poids par rapport au poids total de la composition.

La composition conforme à l'invention comprend au moins un colorant d'oxydation pouvant être choisi parmi les bases d'oxydation et les coupleurs.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques ainsi que les sels d'addition de ces composés avec un acide.

Les para-phénylènediamines utilisables dans le cadre de l'invention peuvent notamment être choisies parmi les composés de formule (IV) suivante et leurs sels d'addition avec un acide : 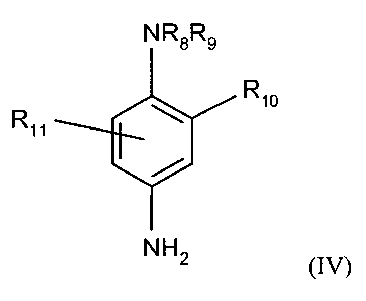 dans laquelle :
R₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
R₉ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₈ et R₉ peuvent également former avec l'atome d'azote qui les porte un hétérocycle azoté à 5 ou 6 chaînons éventuellement substitué par un ou plusieurs groupements alkyle, hydroxy ou uréido ;
R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
R₁₁ représente un atome d'hydrogène, d'halogène ou un radical alkyle en C₁-C₄.

Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (IV) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloroaniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, la 2-méthyl-1-N-β-hydroxyéthyl-paraphénylènediamine, et leurs sels d'addition avec un acide.

Parmi les paraphénylènediamines de formule (IV) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

Selon l'invention, on entend par bases doubles les composés comportant au moins deux noyaux aromatiques sur lesquels sont portés des groupements amino et / ou hydroxyle.

Parmi les bases doubles utilisables à titre de bases d'oxydation dans la composition conforme à l'invention, on peut notamment citer les composés répondant à la formule (V) suivante et leurs sels d'addition avec un acide : 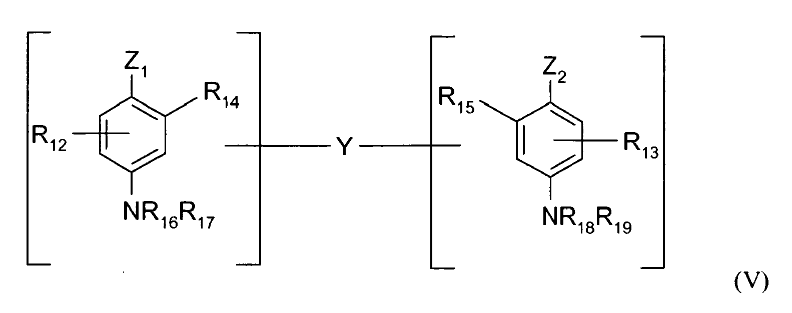 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₄, R₁₅, R₁₆, R₁₇, R₁₈ et R₁₉, identiques ou différents, représentent un atome d'hydrogène, un bras de liaison Y ou un radical alkyle en C₁-C₄ ;
étant entendu que les composés de formule (V) ne comportent qu'un seul bras de liaison Y par molécule.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (V) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (V), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols utilisables dans le cadre de l'invention peuvent notamment être choisis parmi les composés répondant à la formule (VI) suivante et leurs sels d'addition avec un acide : 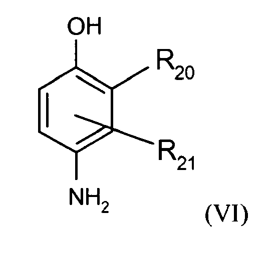 dans laquelle :
R₂₀ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄ ;
R₂₁ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (VI) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

La composition selon l'invention comprend généralement une quantité totale de base(s) d'oxydation allant de 0,0005 à 12 % en poids par rapport au poids total de la composition. De préférence, elle comprend une quantité totale de base(s) d'oxydation allant de 0,005 à 8 % en poids, et mieux encore de 0,05 à 5 % en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La composition selon l'invention comprend généralement une quantité totale de coupleur(s) allant de 0,0001 à 15 % en poids par rapport au poids total de la composition. De préférence, elle comprend une quantité totale de coupleur(s) allant de 0,001 à 10 % en poids, et mieux encore de 0,01 à 8 % en poids, par rapport au poids total de la composition.

D'une manière générale, les sels d'addition avec un acide des bases d'oxydation et des coupleurs sont notamment choisis parmi les chlorhydrates, les bromhydrates, les sulfates et les tartrates, les lactates et les acétates.

Selon un mode de réalisation particulier de l'invention, la composition comprend au moins une base d'oxydation et au moins un coupleur.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition conforme à la présente invention peut comprendre au moins un agent oxydant.

Un tel agent oxydant est choisi de préférence dans le groupe formé par le peroxyde d'hydrogène, le peroxyde d'urée, les bromates ou ferricyanures de métaux alcalins, les persels tels que les perborates et les persulfates.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), éventuellement en présence de leur donneur ou cofacteur respectif.

Le milieu approprié pour la teinture appelé aussi support de teinture est un milieu cosmétique généralement constitué par de l'eau ou par un mélange d'eau et d'au moins un solvant organique acceptable sur le plan cosmétique. A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20 % et, de préférence, entre environ 2 et 10 % en poids par rapport au poids total de la composition.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, autres que les polymères amphotères selon l'invention, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition tinctoriale.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

Le pH de la composition conforme à l'invention est généralement compris entre 3 et 12 environ, de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule suivante : 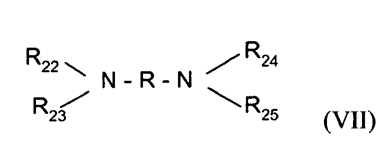 dans laquelle R est un reste propylène éventuellement substitué par un groupement hydroxyle ou un radical alkyle en C₁-C₄ ; R₂₂, R₂₃, R₂₄ et R₂₅, identiques ou différents, représentent un atome d'hydrogène, un radical alkyle en C₁-C₄ ou hydroxyalkyle en C₁-C₄.

Parmi les agents acidifiants, on peut citer, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose variant généralement de 1 à 60 minutes environ, de préférence 5 à 45 minutes environ, les fibres kératiniques sont rincées, lavées au shampooing, rincées à nouveau puis séchées.

L'invention a aussi pour objet un dispositif à plusieurs compartiments ou "kit" de teinture dans lequel un premier compartiment contient une composition tinctoriale selon la présente invention à l'exception de l'agent oxydant, et un deuxième compartiment contient un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la teinture d'oxydation des fibres kératiniques, et en particulier des fibres kératiniques humaines telles que les cheveux, d'une composition tinctoriale telle que définie précédemment.

Les exemples qui suivent sont destinés à illustrer l'invention.

Dans ces exemples, toutes les quantités sont indiquées en pour cent en poids de matière active (M.A.) par rapport au poids total de la composition, sauf indication contraire.

### Exemple 1 comparatif:

On a préparé deux compositions de teinture à partir des composés suivants (composition A comparative, composition B selon l'invention):

| **Composition** | **A** | **B** |
|---|---|---|
| alcool laurique oxyéthyléné à 12 OE commercialisé sous la dénomination REWOPAL 12 par la société GOLDSCHMIDT | 7,5 | 7,5 |
| monostéarate de glycol | 2 | 2 |
| alcool oléocétylique oxyéthyléné à 30 OE commercialisé sous la dénomination EMULGIN O 30 par la société COGNIS | 3 | 3 |
| alcool décylique oxyéthyléné à 3 OE commercialisé sous la dénomination EMULGIN BL 309 par la société COGNIS | 10 | 10 |
| alcool cétylstéarylique (C16/C18 : 50/50) commercialisé sous la dénomination LANETTE O OR par la société COGNIS | 10 | 10 |
| acide laurique naturel | 2,5 | 2,5 |
| silice pyrogénée hydrophobe commercialisée sous la dénomination AEROSIL R972 par la société DEGUSSA | 1 | 1 |
| acide polyacrylique réticulé commercialisé sous la dénomination CARBOPOL 980 par la société NOVEON | 0,4 | 0,4 |
| propylène glycol | 10 | 10 |
| monoéthanolamine | 1,2 | 1,2 |
| polymère acrylamide / chlorure de diallyldiméthyl ammonium / acide acrylique (Merquat 3331 de Nalco) | - | 2,4 M.A. |
| acide diéthylène triamine pentacétique, sel pentasodique en solution aqueuse à 40% commercialisé sous la dénomination DISSOLVINE D40 par la société AKZO NOBEL | 2 | 2 |
| thiolactate d'ammonium en solution aqueuse à 58% (50% en acide thiolactique) | 0,8 | 0,8 |
| acide ascorbique | 0,2 | 0,2 |
| dioxyde de titane | 0,2 | 0,2 |
| ammoniaque à 20% de NH₃ | 10 | 10 |
| 2-méthyl-5-hydroxyéthyl aminophénol | 0,86 | 0,86 |
| p-aminophénol | 0,41 | 0,41 |
| 4-amino-2-hydroxytoluène | 0,57 | 0,57 |
| 6-hydroxyindole | 0,068 | 0,068 |
| p-phénylènediamine | 0,49 | 0,49 |
| résorcinol | 0,1 | 0,1 |
| parfum | qs | qs |
| eau | qsp 100 | qsp 100 |

On a mélangé dans un rapport pondéral 1 + 1,5 chacune des compositions A et B avec une composition oxydante titrant 20 volumes en eau oxygénée.
Les pH des mélanges ainsi obtenus sont de 10.
Ces mélanges sont appliqués sur des cheveux gris à 90 % de blancs pendant un temps de pose de 30 minutes à température ambiante.
Les cheveux sont ensuite rincés, lavés avec un shampooing standard, puis rincés à l'eau et séchés.
On obtient, avec les mélanges issus des compositions A et B avec la composition oxydante, une nuance à reflet cuivré rouge esthétique de bonne ténacité.
On observe que le mélange issu de la composition B avec la composition oxydante engendre moins de phénomènes d'électricité statique à la surface des cheveux traités que celui obtenu avec la composition A.
En outre, après coloration avec le mélange issu de la composition B, on observe que les cheveux sont particulièrement lisses.

## Revendications

1. Composition pour la teinture d'oxydation des fibres kératiniques comprenant, dans un milieu approprié pour la teinture, au moins un colorant d'oxydation et au moins un polymère amphotère comprenant la répétition de :
(i) de 45 à 60 % en moles de un ou plusieurs motifs issus d'un monomère de type acrylamide,
(ii) un ou plusieurs motifs issus d'un monomère de type halogénure de dialkyldiallylammonium, et
(iii) un ou plusieurs motifs issus d'un monomère de type acide carboxylique vinylique,
avec un rapport molaire du taux de motifs issus d'un monomère de type halogénure de dialkyldiallylammonium sur le taux de motifs issus d'un monomère de type acide carboxylique vinylique supérieur ou égal à 1.

2. Composition selon la revendication 1 dans laquelle les motifs issus d'un monomère de type acrylamide sont des motifs de structure (I) suivante : 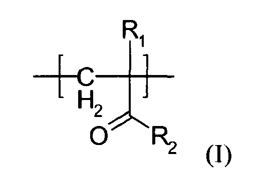 dans laquelle :
- R₁ désigne H ou CH₃,
- R₂ est choisi parmi un radical amino, diméthylamino, tert-butylamino, dodécylamino, ou
- NH-CH₂OH.

3. Composition selon la revendication 2 dans laquelle R₁ désigne H et R₂ est un radical amino.

4. Composition selon l'une quelconque des revendications précédentes dans laquelle les motifs issus d'un monomère de type halogénure de dialkyldiallylammonium sont des motifs de structure (II) suivante : 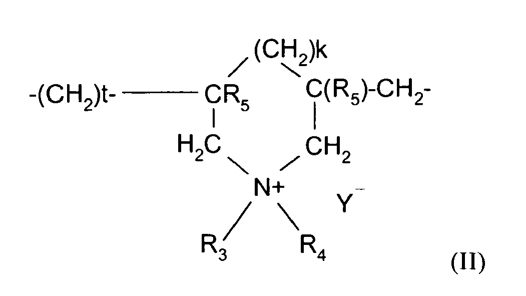 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de 1 à 5 atomes de carbone, un groupement amidoalkyle(C₁-C₄), ou R₃ et R₄ forment conjointement avec l'atome d'azote auquel ils sont rattachés, des groupements hétérocycliques ;
- Y⁻ est un anion bromure, chlorure, acétate, borate, citrate, tartrate, bisulfate, bisulfite, sulfate ou phosphate.

5. Composition selon la revendication 4 dans laquelle les motifs issus d'un monomère de type halogénure de dialkyldiallylammonium sont des motifs de formule (II) dans laquelle R₅ désigne un atome d'hydrogène et R₃ et R₄ désignent un radical méthyle, Y⁻ désignant un anion chlorure.

6. Composition selon l'une quelconque des revendications précédentes dans laquelle les motifs issus d'un monomère de type acide carboxylique vinylique sont choisis parmi les motifs de formule (III) : 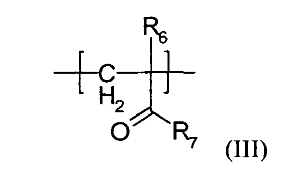 dans laquelle :
- R₆ désigne H ou CH₃,
- R₇ désigne un radical hydroxyle ou un radical -NH-C(CH₃)₂-CH₂-SO₃H.

7. Composition selon la revendication 6 dans laquelle le motif issu d'un monomère de type acide carboxylique vinylique est celui pour lequel R₆ désigne un atome d'hydrogène et R₇ désigne un radical hydroxyle.

8. Composition selon l'une quelconque des revendications précédentes dans laquelle le ou les polymères amphotères comprennent de 45 à 55 % en moles de motifs issus d'un monomère de type acrylamide.

9. Composition selon l'une quelconque des revendications précédentes dans laquelle le ou les polymères amphotères comprennent 30 ou moins de 30% en moles de motifs issus d'un monomère de type acide carboxylique vinylique.

10. Composition selon l'une quelconque des revendications précédentes dans laquelle, dans le ou les polymères amphotères, le rapport molaire du taux de motifs issus d'un monomère de type halogénure de dialkyldiallylammonium sur le taux de motifs issus d'un monomère de type acide carboxylique vinylique est compris entre 1 et 2.

11. Composition selon l'une quelconque des revendications précédentes dans laquelle le ou les polymères amphotères comprennent au moins 25 % en moles de motifs issus d'un monomère de type halogénure de dialkyldiallylammonium.

12. Composition selon l'une quelconque des revendications précédentes dans laquelle le ou les polymères amphotères sont choisis parmi les terpolymères acrylamide / chlorure de diméthyldiallylammonium / acide acrylique.

13. Composition selon l'une quelconque des revendications précédentes dans laquelle le ou les polymères amphotères sont présents en quantité comprise entre 0,1 et 10 % en poids par rapport au poids total de la composition.

14. Composition selon l'une quelconque des revendications précédentes dans laquelle le ou les colorants d'oxydation sont choisis parmi les bases d'oxydation et les coupleurs.

15. Composition selon la revendication 14 dans laquelle la ou les bases d'oxydation sont choisies parmi les ortho- et para-phénylènediamines, les bases doubles, les ortho- et para- aminophénols, les bases hétérocycliques ainsi que les sels d'addition de ces composés avec un acide.

16. Composition selon la revendication 14 ou 15 comprenant une quantité totale de base(s) d'oxydation allant de 0,0005 à 12 % en poids par rapport au poids total de la composition.

17. Composition selon l'une quelconque des revendications 14 à 16 dans laquelle le ou les coupleurs sont choisis parmi les méta-aminophénols, les méta-phénylènediamines, les métadiphénols, les naphtols, les coupleurs hétérocycliques et les sels d'addition de ces composés avec un acide.

18. Composition selon l'une quelconque des revendications 14 à 17 comprenant une quantité totale de coupleurs(s) allant de 0,0001 à 15 % en poids par rapport au poids total de la composition.

19. Composition selon l'une quelconque des revendications précédentes comprenant au moins un agent oxydant.

20. Composition selon la revendication 19 dans laquelle l'agent oxydant est choisi parmi le peroxyde d'hydrogène, le peroxyde d'urée, les bromates ou ferricyanures de métaux alcalins, les persels, les enzymes d'oxydoréduction éventuellement en présence de leur donneur ou cofacteur respectif.

21. Procédé de teinture des fibres kératiniques, **caractérisé en ce qu'**une composition telle que définie à l'une quelconque des revendications 1 à 18 est appliquée sur les fibres kératiniques en présence d'un agent oxydant pendant un temps suffisant pour développer la coloration désirée.

22. Dispositif à plusieurs compartiments dans lequel un premier compartiment contient une composition telle que définie à l'une quelconque des revendications 1 à 18 et un deuxième compartiment contient un agent oxydant.

23. Utilisation pour la teinture d'oxydation des fibres kératiniques d'une composition telle que définie à l'une quelconque des revendications 1 à 20.

## Claims

1. Composition for the oxidation dyeing of keratin fibres, comprising, in a medium that is suitable for dyeing, at least one oxidation dye and at least one amphoteric polymer comprising a repetition of:
(i) from 45 mol% to 60 mol% of one or more units derived from a monomer of acrylamide type,
(ii) one or more units derived from a monomer of dialkyldiallylammonium halide type, and
(iii) one or more units derived from a monomer of vinylcarboxylic acid type,
with a mole ratio of the content of units derived from a monomer of dialkyldiallylammonium halide type to the content of units derived from a monomer of vinylcarboxylic acid type of greater than or equal to 1.

2. Composition according to Claim 1, in which the units derived from a monomer of acrylamide type are units of structure (I) below: 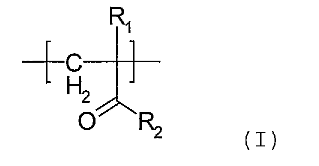 in which:
- R₁ denotes H or CH₃,
- R₂ is chosen from an amino, dimethylamino, tert-butylamino, dodecylamino or -NH-CH₂OH radical.

3. Composition according to Claim 2, in which R₁ denotes H and R₂ is an amino radical.

4. Composition according to any one of the preceding claims, in which the units derived from a monomer of dialkyldiallylammonium halide type are units of structure (II) below: 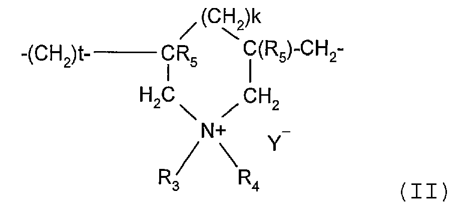 in which:
- k and t are equal to 0 or 1, the sum k + t being equal to 1;
- R₅ denotes a hydrogen atom or a methyl radical;
- R₃ and R₄, independently of each other, denote an alkyl group containing from 1 to 4 carbon atoms, a hydroxyalkyl group in which the alkyl group contains from 1 to 5 carbon atoms, or an amido(C₁-C₄)alkyl group, or R₃ and R₄ form, together with the nitrogen atom to which they are attached, heterocyclic groups;
- Y⁻ is a bromide, chloride, acetate, borate, citrate, tartrate, bisulfate, bisulfite, sulfate or phosphate anion.

5. Composition according to Claim 4, in which the units derived from a monomer of dialkyldiallylammonium halide type are units of formula (II) in which R₅ denotes a hydrogen atom and R₃ and R₄ denote a methyl radical, Y⁻ denoting a chloride anion.

6. Composition according to any one of the preceding claims, in which the units derived from a monomer of vinylcarboxylic acid type are chosen from the units of formula (III): 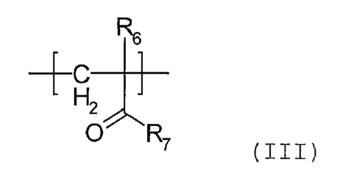 in which:
- R₆ denotes H or CH₃,
- R₇ denotes a hydroxyl radical or a radical -NH-C (CH₃)₂-CH₂-SO₃H.

7. Composition according to Claim 6, in which the unit derived from a monomer of vinylcarboxylic acid type is that for which R₆ denotes a hydrogen atom and R₇ denotes a hydroxyl radical.

8. Composition according to any one of the preceding claims, in which the amphoteric polymer(s) comprise(s) from 45 mol% to 55 mol% of units derived from a monomer of acrylamide type.

9. Composition according to any one of the preceding claims, in which the amphoteric polymer(s) comprise 30 mol% or less than 30 mol% of units derived from a monomer of vinylcarboxylic acid type.

10. Composition according to any one of the preceding claims, in which, in the amphoteric polymer(s), the mole ratio of the content of units derived from a monomer of dialkyldiallylammonium halide type to the content of units derived from a monomer of vinylcarboxylic acid type is between 1 and 2.

11. Composition according to any one of the preceding claims, in which the amphoteric polymer(s) comprise(s) at least 25 mol% of units derived from a monomer of dialkyldiallylammonium halide type.

12. Composition according to any one of the preceding claims, in which the amphoteric polymer(s) is (are) chosen from acrylamide/dimethyldiallylammonium chloride/acrylic acid terpolymers.

13. Composition according to any one of the preceding claims, in which the amphoteric polymer(s) is (are) present in an amount of between 0.1% and 10% by weight relative to the total weight of the composition.

14. Composition according to any one of the preceding claims, in which the oxidation dye(s) is (are) chosen from oxidation bases and couplers.

15. Composition according to Claim 14, in which the oxidation base(s) is (are) chosen from ortho- and paraphenylenediamines, double bases, ortho- and paraaminophenols and heterocyclic bases, and also the addition salts of these compounds with an acid.

16. Composition according to Claim 14 or 15, comprising a total amount of oxidation base(s) ranging from 0.0005% to 12% by weight relative to the total weight of the composition.

17. Composition according to any one of Claims 14 to 16, in which the coupler(s) is (are) chosen from metaaminophenols, meta-phenylenediamines, meta-diphenols, naphthols and heterocyclic couplers, and the addition salts of these compounds with an acid.

18. Composition according to any one of Claims 14 to 17, comprising a total amount of coupler(s) ranging from 0.0001% to 15% by weight relative to the total weight of the composition.

19. Composition according to any one of the preceding claims, comprising at least one oxidizing agent.

20. Composition according to Claim 19, in which the oxidizing agent is chosen from hydrogen peroxide, urea peroxide, alkali metal bromates or ferricyanides, persalts, and redox enzymes optionally in the presence of the respective donor or cofactor thereof.

21. Process for dyeing keratin fibres, **characterized in that** a composition as defined in any one of Claims 1 to 18 is applied to the keratin fibres in the presence of an oxidizing agent for a time that is sufficient to develop the desired coloration.

22. Multi-compartment device in which a first compartment contains a composition as defined in any one of Claims 1 to 18 and a second compartment contains an oxidizing agent.

23. Use, for the oxidation dyeing of keratin fibres, of a composition as defined in any one of Claims 1 to 20.

## Patentansprüche

1. Zusammensetzung zum Oxidationsfärben von Keratinfasern, umfassend in einem für die Färbung geeigneten Medium mindestens ein Oxidationsfärbemittel und mindestens ein amphoteres Polymer mit der Wiederholung:
(i) von 45 bis 60 Mol-% einer oder mehrerer Einheiten, die sich von einem Monomer vom Acrylamid-Typ ableiten,
(ii) einer oder mehrerer Einheiten, die sich von einem Monomer vom Dialkyldiallylammoniumhalogenid-Typ ableiten, und
(iii) einer oder mehrerer Einheiten, die sich von einem Monomer vom Vinylcarbonsäure-Typ ableiten, mit einem Molverhältnis des Gehalts an Einheiten, die sich von einem Monomer vom Dialkyldiallylammoniumhalogenid-Typ ableiten, zum Gehalt an Einheiten, die sich von einem Monomer vom Vinylcarbonsäure-Typ ableiten, größer oder gleich 1.

2. Zusammensetzung nach Anspruch 1, wobei es sich bei den Einheiten, die sich von einem Monomer vom Acrylamid-Typ ableiten, um Einheiten der folgenden Struktur (I) handelt: 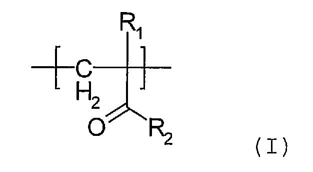 worin:
- R₁ für H oder CH₃ steht,
- R₂ unter einem Amino-, Dimethylamino-, tert.-Butylamino-, Dodecylamino- oder -NH-CH₂OH-Res ausgewählt ist.

3. Zusammensetzung nach Anspruch 2, wobei R₁ für H steht und R₂ für einen Aminorest steht.

4. Zusammensetzung nach einem der vorhergehenden Ansprüche, wobei es sich bei den Einheiten, die sich von einem Monomer vom Dialkyldiallylammoniumhalogenid-Typ ableiten, um Einheiten der folgenden Struktur (II) handelt: 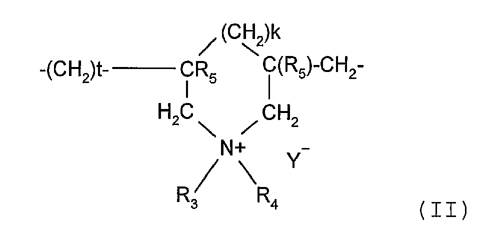 worin:
- k und t gleich 0 oder 1 sind, wobei die Summe k + t gleich 1 ist;
- R₅ für ein Wasserstoffatom oder einen Methylrest steht;
- R₃ und R₄ unabhängig voneinander für eine Alkylgruppe mit 1 bis 4 Kohlenstoffatomen, eine Hydroxyalkylgruppe mit 1 bis 5 Kohlenstoffatomen in der Alkylgruppe oder eine Amido(C₁-C₄)alkylgruppe stehen oder R₃ und R₄ zusammen mit dem Stickstoffatom, an das sie gebunden sind, heterocyclische Gruppen bilden;
- Y⁻ für ein Bromid-, Chlorid-, Acetat-, Borat-, Citrat-, Tartrat-, Hydrogensulfat-, Hydrogensulfit-, Sulfat- oder Phosphatanion steht.

5. Zusammensetzung nach Anspruch 4, wobei es sich bei den Einheiten, die sich von einem Monomer vom Dialkyldiallylammoniumhalogenid-Typ ableiten, um Einheiten der Struktur (II), worin R₅ für ein Wasserstoffatom steht und R₃ und R₄ für einen Methylrest stehen, wobei Y⁻ für ein Chlorid-Anion steht, handelt.

6. Zusammensetzung nach einem der vorhergehenden Ansprüche, wobei die Einheiten, die sich von einem Monomer vom Vinylcarbonsäure-Typ ableiten, unter den Einheiten der Formel (III) ausgewählt sind: 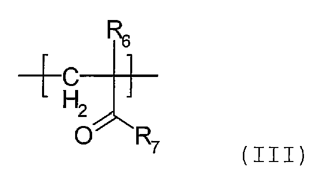 worin:
- R₆ für H oder CH₃ steht,
- R₇ für einen Hydroxylrest oder einen -NH-C(CH₃)₂-CH₂-SO₃H-Rest steht.

7. Zusammensetzung nach Anspruch 6, wobei es sich bei der Einheit, die sich von einem Monomer vom Vinylcarbonsäure-Typ ableitet, um diejenige handelt, für die R₆ für ein Wasserstoffatom steht und R₇ für einen Hydroxylrest steht.

8. Zusammensetzung nach einem der vorhergehenden Ansprüche, wobei das oder die amphoteren Polymere 45 bis 55 Mol-% Einheiten, die sich von einem Monomer vom Acrylamid-Typ ableiten, umfassen.

9. Zusammensetzung nach einem der vorhergehenden Ansprüche, wobei das oder die amphoteren Polymere 30 oder weniger als 30 Mol-% Einheiten, die sich von einem Monomer vom Vinylcarbonsäure-Typ ableiten, umfassen.

10. Zusammensetzung nach einem der vorhergehenden Ansprüche, wobei in dem oder den amphoteren Polymeren das Molverhältnis des Gehalts an Einheiten, die sich von einem Monomer vom Dialkyldiallylammoniumhalogenid-Typ ableiten, zum Gehalt an Einheiten, die sich von einem Monomer vom Vinylcarbonsäure-Typ ableiten, zwischen 1 und 2 liegt.

11. Zusammensetzung nach einem der vorhergehenden Ansprüche, wobei das oder die amphoteren Polymere mindestens 25 Mol-% Einheiten, die sich von einem Monomer vom Dialkyldiallylammoniumhalogenid-Typ ableiten, umfassen.

12. Zusammensetzung nach einem der vorhergehenden Ansprüche, wobei das oder die amphoteren Polymere unter Acrylamid/Dimethyldiallylammoniumchlorid/Acrylsäure-Terpolymeren ausgewählt sind.

13. Zusammensetzung nach einem der vorhergehenden Ansprüche, wobei das oder die amphoteren Polymere in einer Menge zwischen 0,1 und 10 Gew.-%, bezogen auf das Gesamtgewicht der Zusammensetzung, vorliegen.

14. Zusammensetzung nach einem der vorhergehenden Ansprüche, wobei das oder die Oxidationsfärbemittel unter Oxidationsbasen und Kupplern ausgewählt sind.

15. Zusammensetzung nach Anspruch 14, wobei die Oxidationsbase(n) unter ortho- und paraPhenylendiaminen, Doppelbasen, ortho- und para-Aminophenolen, heterocyclischen Basen und Additionssalzen dieser Verbindungen mit einer Säure ausgewählt sind.

16. Zusammensetzung nach Anspruch 14 oder 15, umfassend eine Gesamtmenge an Oxidationsbase(n) von 0,0005 bis 12 Gew.-%, bezogen auf das Gesamtgewicht der Zusammensetzung.

17. Zusammensetzung nach einem der Ansprüche 14 bis 16, wobei der oder die Kuppler unter meta-Aminophenolen, meta-Phenylendiaminen, meta-Diphenolen, Naphtholen, heterocyclischen Kupplern und Additionssalzen dieser Verbindungen mit einer Säure ausgewählt sind.

18. Zusammensetzung nach einem der Ansprüche 14 bis 17, umfassend eine Gesamtmenge an Kuppler(n) von 0,0001 bis 15 Gew.-%, bezogen auf das Gesamtgewicht der Zusammensetzung.

19. Zusammensetzung nach einem der vorhergehenden Ansprüche, umfassend mindestens ein Oxidationsmittel.

20. Zusammensetzung nach Anspruch 19, wobei das Oxidationsmittel unter Wasserstoffperoxid, Harnstoffperoxid, Alkalimetallbromaten oder -ferricyaniden, Persalzen und Redoxenzymen, gegebenenfalls in Gegenwart ihres jeweiligen Donors oder Cofaktors, ausgewählt ist.

21. Verfahren zum Färben von Keratinfasern, **dadurch gekennzeichnet, dass** man auf die Keratinfasern eine Zusammensetzung gemäß einem der Ansprüche 1 bis 18 in Gegenwart eines Oxidationsmittels über einen Zeitraum, der zur Entwicklung der gewünschten Färbung ausreicht, aufbringt.

22. Vorrichtung mit mehreren Kompartimenten, wobei ein erstes Kompartiment eine Zusammensetzung gemäß einem der Ansprüche 1 bis 18 enthält und ein zweites Kompartiment ein Oxidationsmittel enthält.

23. Verwendung einer Zusammensetzung gemäß einem der Ansprüche 1 bis 20 zum Oxidationsfärben von Keratinfasern.
